# EUROPÄISCHE PATENTSCHRIFT

(11) **EP 4 361 123 B1**
(45) Veröffentlichungstag und Bekanntmachung des Hinweises auf die Patenterteilung: **29.01.2025**
(21) Anmeldenummer: 22203329.2
(22) Anmeldetag: 24.10.2022
(51) Int. Cl.: C07C 45/50, C07C 47/02

(54) **KATALYSATORPRÄFORMIERUNG IN DER HYDROFORMYLIERUNG**
CATALYST PREFORMATION IN HYDROFORMYLATION
PRÉFORMATION DE CATALYSEUR DANS L'HYDROFORMYLATION

(43) Veröffentlichungstag der Anmeldung: 01.05.2024
(73) Patentinhaber: Evonik Oxeno GmbH & Co. KG, 45772 Marl (DE)
(72) Erfinder: GOTTSCHALK, Axel, 45527 Hattingen (DE); TLATLIK, Stephen, 44309 Dortmund (DE)
(74) Vertreter: Evonik Patent Association

(56) Entgegenhaltungen:
- WO-A1-93/24436
- US-A- 5 237 105

## Beschreibung

Die vorliegende Erfindung betrifft ein Hydroformylierungsverfahren, bei dem ein Olefin mit 2 bis 20 Kohlenstoffatomen mit Synthesegas in Gegenwart eines Hydroformylierungskatalysators in mindestens einem Hydroformylierungsreaktor umgesetzt wird, wobei das Verfahren eine Katalysatorpräformierung umfasst, bei der ein Teil einer flüssigen Reaktionsmischung aus dem mindestens einen Hydroformylierungsreaktor entnommen, in einem Präformierungsgefäß mit Kohlenmonoxid in Kontakt gebracht wird und zurück zum Hydroformylierungsreaktor geführt wird. Die Hydroformylierung stellt ein technisch bedeutsames Verfahren zur Gewinnung chemischer Grundstoffe dar, die oftmals in weiteren Synthesen zu Zielprodukten verarbeitet werden. Bei der Hydroformylierung werden Olefine mit Synthesegas, einem Gemisch aus hauptsächlich Kohlenstoffmonoxid und Wasserstoff, in Gegenwart eines homogenen Katalysatorsystems zu Aldehyden umgesetzt. Diese Umsetzung erfolgt durch Einleitung der gasförmigen Reaktanden Kohlenstoffmonoxid und Wasserstoff in ein flüssiges Reaktionsgemisch enthaltend das Olefin und ein homogen darin verteiltes Katalysatorsystem aus einem Metall-Liganden-System. Typischerweise werden Kobalt- oder Rhodiumkatalysatoren eingesetzt. Das Dokument US 5 237 105 offenbart ein Verfahren zur Hydroformylierung von Olefinen unter Verwendung von Synthesegas.

Da katalytische Metall-Liganden-Systeme, welche als Katalysatorsysteme in der Hydroformylierung eingesetzt werden können, oftmals verschiedene zielführende und nicht zielführende Modifikationen ausbilden, wird üblicherweise mittels eines Präformierungsschrittes angestrebt, die aktive, d.h. die gewünschte Modifikation möglichst vollständig zu erhalten. Dies gilt sowohl für Katalysatoren, die zu Beginn oder während der Reaktion frisch in den Reaktor eingetragen werden, als auch für bereits im Prozess befindliche Katalysatorkomplexe, die durch thermische, chemische oder andere Belastungen unerwünschte Spezies ausgebildet haben können.

Die vorliegende Erfindung hat zur Aufgabe ein vorteilhaftes Verfahren zur Katalysatorpräformierung bereitzustellen. Da es sich bei der Präformierung um eine chemische Reaktion von Katalysatorsystem und Kohlenstoffmonoxid handelt, existieren auch für diesen Verfahrensschritt optimale Reaktionsbedingungen, die die Präformierung besonders vorteilhaft verlaufen lassen. Diese optimalen Präformierungsbedingungen decken sich allerdings nicht zwingend mit den Betriebsbedingungen des Hydroformylierungsprozesses, sodass die kontinuierliche Aufbereitung des Katalysators gegebenenfalls separat erfolgen muss, was einen erheblichen energetischen und apparativen Aufwand erfordert. Diese Nachteile sollen durch die vorliegende Erfindung umgangen werden.

Im Rahmen der vorliegenden Erfindung bezeichnet der Begriff Hydroformylierungsverfahren ein Verfahren zur Herstellung von Aldehyden, wobei ein Olefin mit Kohlenstoffmonoxid und Wasserstoff in einer flüssigen Reaktionsmischung in Gegenwart eines homogenen Katalysatorsystems zu einem Aldehyd umgesetzt wird.

Die vorliegende Erfindung betrifft ein Hydroformylierungsverfahren, bei dem ein Olefin mit 4 bis 20 Kohlenstoffatomen mit Synthesegas in Gegenwart eines Hydroformylierungskatalysators in mindestens einem Hydroformylierungsreaktor umgesetzt wird, wobei das Verfahren die folgenden Schritte umfasst
a) ein Teil einer flüssigen Reaktionsmischung enthaltend einen Hydroformylierungskatalysator aus einem Hydroformylierungsreaktor entnommen wird,
b) die entnommene flüssige Reaktionsmischung in ein Präformierungsgefäß überführt wird,
c) die flüssige Reaktionsmischung in dem Präformierungsgefäß mit Kohlenstoffmonoxid in Kontakt gebracht wird, und
d) die flüssige Reaktiosmischung wieder in den Hydroformylierungsreaktor zurückgeführt wird.

Durch das erfindungsgemäße Verfahren ist es möglich, den Hydroformylierungskatalysator unabhängig von den Reaktionsbedingungen im Hydroformylierungsreaktor zu präformieren, wobei die Präformierungsbedingungen frei wählbar sind und die Katalysatorpräformierung in einem kontinuierlichen Prozess erfolgen kann. Der Vorteil des erfindungsgemäßen Verfahrens liegt in der apparativen und steuerungstechnischen Einfachheit des Aufbaus. Das Verfahren lässt sich so beispielsweise auch durch Nachrüstung bestehender Hydroformylierungsanlagen leicht implementieren.

### Hydroformylierung

Bei der Hydroformylierung nach dem vorliegenden Verfahren werden Olefine mit 2 bis 20 Kohlenstoffatomen umgesetzt. Vorzugsweise werden Olefine mit 4 bis 16 Kohlenstoffatomen, besonders bevorzugt Olefine mit 4 bis 12 Kohlenstoffatomen eingesetzt. Die eingesetzten Feedströme sind üblicherweise technisch verfügbare Kohlenwasserstoffströme, die zumindest verschiedene Isomere des jeweiligen Olefins enthalten. Die Feedströme enthalten dabei zumindest lineare Olefine und verzweigte Olefine mit jeweils gleicher Anzahl an Kohlenstoffatomen. Die Menge an den jeweiligen Olefinen in den Kohlenwasserstoffströmen sollte verständlicherweise ausreichend hoch sein, um die Oxierung in Schritt c) wirtschaftlich betreiben zu können.

Feedströme, die Olefine mit 4 Kohlenstoffatomen enthalten, beispielsweise Mischungen der verschiedenen Isomere des Butens (1-Buten, 2-Buten, Isobuten), sind üblicherweise Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische mit teils unterschiedlicher Konzentration an den Isomeren. Beispielsweise können für das erfindungsgemäße Verfahren geeignete Gemische linearer Butene aus der C4-Fraktion eines Steamcrackers gewonnen werden. Olefine mit 5 Kohlenstoffatomen, also Pentene, sind in Leichtbenzinfraktionen aus Raffinerien oder Crackern enthalten.

Die höheren Olefine können insbesondere durch Oligomerisierungsreaktionen, beispielsweise Dimerisierung, Trimerisierung oder Tetramerisierung, gewonnen werden. Geeignete Kohlenwasserstoffströme sind weiterhin das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Die entsprechenden Olefine werden mit Synthesegas umgesetzt. Synthesegas ist erfindungsgemäß eine Mischung aus Wasserstoff und Kohlenmonoxid. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen.

Das bei der Hydroformylierung einsetzbare homogene Katalysatorsystem kann Co oder Rh, vorzugsweise Co und einem phosphorhaltigen Liganden umfassen. Entsprechende Katalysatorsysteme sind dem Fachmann geläufig. Geeignete Liganden für die erfindungsgemäßen Katalysatorsysteme sind dem Fachmann bekannt. Liganden, die sich für das vorliegende Verfahren eignen, sind an verschiedneen Stellen bereits beschrieben worden, z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke. Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der Hydroformylierung liegt vorzugsweise im Bereich von 70 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 20 bis 350 bar, weiterhin bevorzugt im Bereich von 30 bis 325 bar und besonders bevorzugt im Bereich von 45 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck.

Die Hydroformylierung wird weiterhin in mindestens einem Hydroformylierungsreaktor durchgeführt, in dem eine Reaktionsmischung vorliegt. Es ist grundsätzlich möglich mehrere Reaktoren einzusetzen und diese in Reihe oder parallel zu schalten. Eine Beschränkung für das vorliegende Verfahren ergibt sich nicht.

### Präformierung

Das erfindungsgemäße Verfahren sieht auch vor, dass bei Verwendung von mehr als einem Präformierungsgefäß diese beliebig bezüglich Gas- und Flüssigphase verschaltet sein können.

Grundsätzlich können Druck und Temperatur im Präformierungsgefäß können bei dem erfindungsgemäßen Hydroformylierungsverfahren grundsätzlich dem Druck und der Temperatur der Hydroformylierung entsprechen. Die Temperatur im Präformierungsgefäß liegt vorzugsweise im Bereich von 70 bis 250 °C, weiterhin bevorzugt im Bereich von 90 bis 225 °C und besonders bevorzugt im Bereich von 100 bis 210 °C. Der Druck im Präformierungsgefäß liegt vorzugsweise im Bereich von 20 bis 350 bar, weiterhin bevorzugt im Bereich von 30 bis 325 bar und besonders bevorzugt im Bereich von 45 bis 300 bar.

In einer Ausführungsform des vorliegenden Verfahrens sind Druck und/oder Temperatur in dem Präformierungsgefäß unterschiedlich zu dem in dem/den Hydroformylierungsreaktor(en) vorliegenden Druck oder der vorliegenden Temperatur. Temperatur und Druck können dabei höher oder niedriger sein als im Hydroformylierungsreaktor, vorzugsweise sind Temperatur und Druck geringer. In dieser Ausführungsform können Druck und Temperatur der flüssigen Reaktionsmischung bei der Überführung in das Präformierungsgefäß und/oder bei der Rückführung in den Hydroformylierungsreaktor jeweils angepasst werden. Dies erfolgt beispielsweise durch Aufheizen bzw. Kühlen bei der Anpassung der Temperatur bzw. durch Entspannen oder Komprimieren beim Anpassen des Drucks. Geeignete verfahrenstechnische An-oder Einbauten, um ein Kühlen / Heizen oder ein Komprimieren / Entspannen zu erreichen sind dem Fachmann geläufig.

Hierdurch ist es möglich, die den Reaktor entnommene Reaktionsmischung hinsichtlich Temperatur und Druck so zu konditionieren, dass in dem Präformierungsgefäß der Kontakt mit Kohlenstoffmonoxid bei optimalen Betriebsbedingungen hergestellt werden kann.

In einer Ausführungsform des Verfahrens erfolgen Entnahme und Rückführung der flüssigen Reaktionsmischung kontinuierlich. Hierdurch sind eine kontinuierliche Katalysatorpräformierung und ein kontinuierlicher Betrieb des Hydroformylierungsprozesses gewährleistet.

In einer Ausführungsform des Verfahrens wird dem Präformierungsgefäß Kohlenstoffmonoxid zugeführt. Hierdurch wird der Verbrauch dieser Komponente durch die Präformierungsreaktion und gegebenenfalls durch die Reaktion während der Hydroformylierung ausgeglichen.

In einer weiteren Ausführungsform des Verfahrens, wird wenigstens ein Teil der Gasphase aus dem Präformierungsgefäß entnommen und dem Hydroformylierungsreaktor zugeführt, wobei Temperatur und/oder Druck der entnommenen Gasphase gegebenenfalls angepasst werden. Die Anpassung kann durch Kühlen / Heizen bzw. durch Komprimieren / Entspannen erfolgen. Geeignete verfahrenstechnische An- oder Einbauten, um ein Kühlen / Heizen oder ein Komprimieren / Entspannen zu erreichen sind dem Fachmann geläufig.

Hierdurch wird der mögliche Nachteil vermieden, dass ein Verdampfen von Komponenten der in das Präformierungsgefäß eingebrachten Reaktionsmischung das Kohlenstoffmonoxid in dem Präformierungsgefäß verdünnt. Stellt sich hierbei kein vorteilhaftes Gleichgewicht zwischen der Verdampfung und der Kondensation beteiligter Komponenten ein, könnte das Kohlenstoffmonoxid vollständig aus dem Präformierungsgefäß verdrängt werden, womit die Präformierung zum Erliegen käme. Diese Gefährdung geht - aus Mangel einer Senke für diese Komponenten - besonders von im Reaktionsgemisch gelösten nicht kondensierbaren Gasen wie zum Beispiel Stickstoff, Argon, Methan oder Kohlenstoffdioxid aus, da diese nur gelöst im Flüssigkeitsstrom das Präformierungsgefäß verlassen können. Durch Entnahme der Gasphase aus dem Präformierungsgefäß wird eine solche Ansammlung flüchtiger Komponenten jedoch vermieden.

In einer weiteren Ausführungsform der vorliegenden Erfindung wird die entnommene Gasphase mit Synthesegas vereinigt, bevor sie dem Hydroformylierungsreaktor zugeführt wird. Die zusätzliche Menge an Wasserstoff und Kohlenstoffmonoxid, die auf diesem Wege in den Hydroformylierungsreaktor gelangt, wird gegebenenfalls durch Anpassung der Menge und der Zusammensetzung des Synthesegasstromes ausgeglichen, um die Konzentration von Wasserstoff und Kohlenstoffmonoxid im Reaktor konstant zu halten bzw. anzupassen.

In einer weiteren Ausführungsform des Verfahrens, wird das dem Präformierungsgefäß zugeführte Kohlenstoffmonoxid zunächst durch Verwendung einer Membran aus dem Synthesegasstrom separiert. Dabei wird aus dem Synthesegasstrom entweder durch Verwendung einer Membran, die eine hohe Selektivität für den Durchgang von Kohlenstoffmonoxid aufweist, ein Permeat erzeugt, welches eine erhöhte Konzentration von Kohlenstoffmonoxid aufweist. Dieses Permeat wird anschließend dem Präformierungsgefäß zugeführt. Alternativ wird aus dem Synthesegasstrom durch Verwendung einer Membran, die eine hohe Selektivität für den Durchgang von Wasserstoff aufweist, ein Retentat erzeugt, welches eine erhöhte Konzentrationen Kohlenstoffmonoxid aufweist. Dieses Retentat kann anschließend dem Präformierungsgefäß zugeführt werden. In beiden Fällen werden gegebenenfalls Druck und/oder Temperatur des Kohlenstoffmonoxids vor der Einleitung in das Präformierungsgefäß angepasst, um optimale Präformierungsbedingungen zu gewährleisten. Bevorzugt wird dem Präformierungsgefäß auch in dieser Ausführungsform ein Teil der Gasphase entnommen und unter Anpassung der Temperatur und/oder des Druckes dem Hydroformylierungsreaktor wie oben beschrieben zugeführt.

Die vorliegende Erfindung wird nachfolgend anhand von Abbildungen illustriert. Diese Abbildungen zeigen jeweils spezielle Ausführungsformen, die die vorliegende Lehre nicht beschränken sollen.

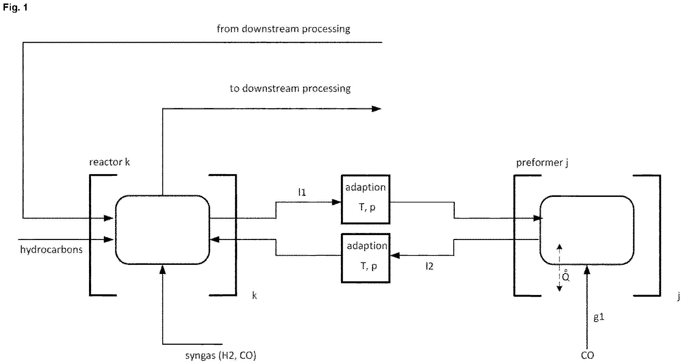

Figur 1 illustriert das erfindungsgemäße Verfahren, wobei dem Hydroformylierungsreaktor k, in dem sich das Reaktionsgemisch inklusive des homogenen Katalysatorsystem befindet und dem Synthesegas zugeführt wird, ein Teil des flüssigen Reaktionsgemisches als Flüssigkeitsteilstrom 11 unter möglicher Anpassung von Temperatur und Druck entnommen und dem Präformierungsgefäß j zugeführt wird. Mit Gasstrom g1 wird dem Präformierungsgefäß der zusätzliche Kohlenstoffmonoxid zugeführt. Im direkten Kontakt des in dem Flüssigkeitsteilstrom I1 enthaltenen Katalysators mit dem Kohlenstoffmonoxid findet die Präformierung statt. Die flüssige Phase der Präformierungsreaktion wird anschließend mittels des Flüssigkeitsstroms I2 unter möglicher Anpassung von Druck und Temperatur in den Reaktor k zurückgeführt.

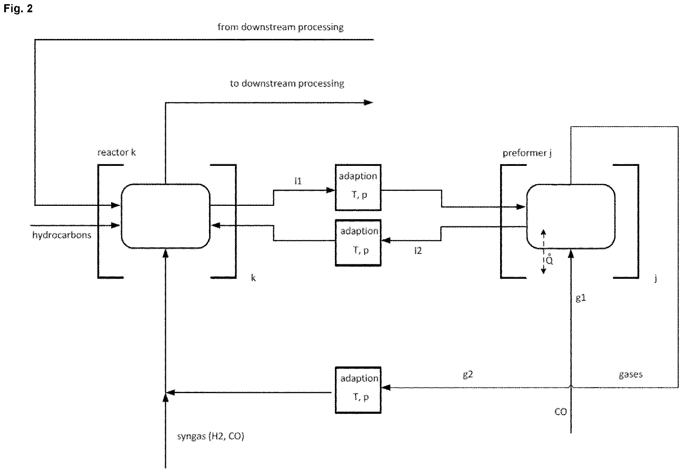

Figur 2 zeigt eine Variante des erfindungsgemäßen Verfahrens, welches in Figur 1 gezeigt ist. Dabei wird mit dem Gasstrom g2 ein Teil der Gasphase aus dem Präformierungsgefäß j entnommen, unter möglicher Anpassung von Druck und Temperatur mit dem Synthesegasstrom vereinigt und dem Reaktor k zugeführt.

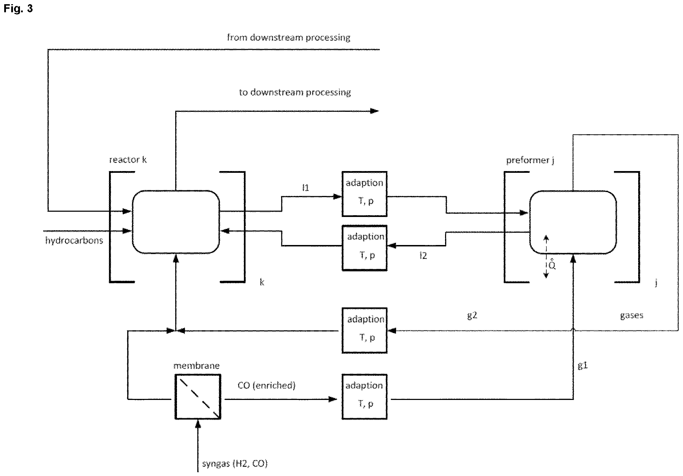

Figur 3 zeigt eine Variante des erfindungsgemäßen Verfahrens, welches in Figur 2 gezeigt ist. Dabei wird durch Verwendung einer Membran, die eine erhöhte Durchlässigkeit für Kohlenstoffmonoxid aufweist, ein Kohlenstoffmonoxid-haltiger Gasstrom g1 aus dem Synthesegasstrom erzeugt, der unter möglicher Anpassung von Druck und Temperatur dem Präformierungsgefäß j zugeführt wird. Das restliche Synthesegas aus der Membran und die Gasphase aus dem Präformierungsgefäß j werden vereinigt und bilden das Synthesegas, welches dem Reaktor k für die Hydroformylierung zugeführt wird.

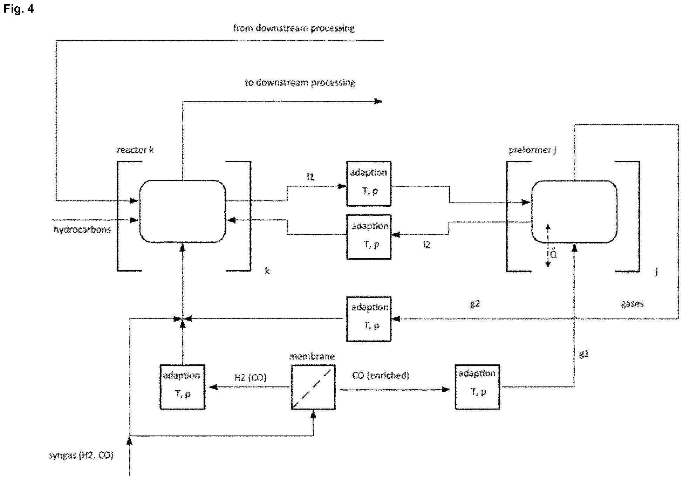

Figur 4 zeigt eine Variante des erfindungsgemäßen Verfahrens, welches in Figur 3 gezeigt ist. Dabei wird durch Verwendung einer Membran, die eine erhöhte Durchlässigkeit für Wasserstoff aufweist, aus dem Synthesegasstrom ein Kohlenstoffmonoxid-haltiger Gasstrom g1 erzeugt, der unter möglicher Anpassung von Druck und Temperatur dem Präformierungsgefäß zugeführt wird. Der restliche an Wasserstoff-angereicherte Strom aus der Membran wird mit weiterem Synthesegas und der Gasphase aus dem Präformierungsgefäß j entnommenen Gasphase vereinigt und als Synthesegas zum Reaktor k für die Hydroformylierung zugeführt.

## Patentansprüche

1. Hydroformylierungsverfahren, bei dem ein Olefin mit 2 bis 20 Kohlenstoffatomen mit Synthesegas in Gegenwart eines Hydroformylierungskatalysators in mindestens einem Hydroformylierungsreaktor umgesetzt wird, wobei das Verfahren die folgenden Schritte umfasst:
a) ein Teil einer flüssigen Reaktionsmischung enthaltend einen Hydroformylierungskatalysator aus einem Hydroformylierungsreaktor entnommen wird,
b) die entnommene flüssige Reaktionsmischung in ein Präformierungsgefäß überführt wird,
c) die flüssige Reaktionsmischung in dem Präformierungsgefäß mit Kohlenstoffmonoxid in Kontakt gebracht wird, und
d) die flüssige Reaktionsmischung wieder in den Hydroformylierungsreaktor zurückgeführt wird.

2. Hydroformylierungsverfahren nach Anspruch 1, wobei Druck und/oder Temperatur in dem Präformierungsgefäß und Druck und/oder Temperatur in dem mindestens einen Hydroformylierungsreaktor unterschiedlich sind.

3. Hydroformylierungsverfahren nach Anspruch 2, wobei Druck und Temperatur der flüssigen Reaktionsmischung bei der Überführung in das Präformierungsgefäß und/oder bei der Rückführung in den Hydroformylierungsreaktor jeweils angepasst werden.

4. Hydroformylierungsverfahren nach einem der vorhergehenden Ansprüche, wobei wenigstens ein Teil der Gasphase aus dem Präformierungsgefäß entnommen und dem Hydroformylierungsreaktor zugeführt wird.

5. Hydroformylierungsverfahren nach Anspruch 4, wobei die entnommene Gasphase mit Synthesegas vereinigt wird, bevor sie dem Hydroformylierungsreaktor zugeführt wird.

6. Hydroformylierungsverfahren nach einem der vorhergehenden Ansprüche, wobei der Hydroformylierungskatalysator Co oder Rh, vorzugsweise Co und einem phosphorhaltigen Liganden umfasst.

7. Hydroformylierungsverfahren nach Anspruch 6, wobei der Hydroformylierungskatalysator Co und einem phosphorhaltigen Liganden umfasst

8. Hydroformylierungsverfahren nach einem der vorhergehenden Ansprüche, wobei die Temperatur bei der Hydroformylierung im Bereich von 70 bis 250 °C.

9. Hydroformylierungsverfahren nach einem der vorhergehenden Ansprüche, wobei der Druck bei der Hydroformylierung im Bereich von 20 bis 350 bar liegt.

10. Hydroformylierungsverfahren nach einem der vorhergehenden Ansprüche, wobei bei dem Verfahren Olefine mit 4 bis 16 Kohlenstoffatomen umgesetzt werden.

11. Hydroformylierungsverfahren nach Anspruch 10, wobei bei dem Verfahren Olefine mit 4 bis 12 Kohlenstoffatomen umgesetzt werden.

## Claims

1. Hydroformylation process in which an olefin having 2 to 20 carbon atoms is reacted with synthesis gas in the presence of a hydroformylation catalyst in at least one hydroformylation reactor, wherein the process comprises the following steps:
a) a portion of a liquid reaction mixture containing a hydroformylation catalyst is withdrawn from a hydroformylation reactor,
b) the withdrawn liquid reaction mixture is transferred to a preforming vessel,
c) the liquid reaction mixture is contacted with carbon monoxide in the preforming vessel, and
d) the liquid reaction mixture is returned to the hydroformylation reactor.

2. Hydroformylation process according to Claim 1, wherein pressure and/or temperature in the preforming vessel and pressure and/or temperature in the at least one hydroformylation reactor are different.

3. Hydroformylation process according to Claim 2, wherein pressure and temperature of the liquid reaction mixture are adjusted in each case on transfer into the preforming vessel and/or on recycling into the hydroformylation reactor.

4. Hydroformylation process according to any of the preceding claims, wherein at least a portion of the gas phase is withdrawn from the preforming vessel and fed to the hydroformylation reactor.

5. Hydroformylation process according to Claim 4, wherein the withdrawn gas phase is combined with synthesis gas before it is fed to the hydroformylation reactor.

6. Hydroformylation process according to any of the preceding claims, wherein the hydroformylation catalyst comprises Co or Rh, preferably Co and a phosphorus-containing ligand.

7. Hydroformylation process according to Claim 6, wherein the hydroformylation catalyst comprises Co and a phosphorus-containing ligand.

8. Hydroformylation process according to any of the preceding claims, wherein the temperature in the hydroformylation is in the range from 70°C to 250°C.

9. Hydroformylation process according to any of the preceding claims, wherein the pressure in the hydroformylation is in the range of 20 to 350 bar.

10. Hydroformylation process according to any of the preceding claims, wherein olefins having 4 to 16 carbon atoms are reacted in the process.

11. Hydroformylation process according to Claim 10, wherein olefins having 4 to 12 carbon atoms are reacted in the process.

## Revendications

1. Procédé d'hydroformylation, dans lequel une oléfine ayant de 2 à 20 atomes de carbone est mise à réagir avec du gaz de synthèse en présence d'un catalyseur d'hydroformylation dans au moins un réacteur d'hydroformylation, le procédé comprenant les étapes suivantes :
a) une partie d'un mélange réactionnel liquide contenant un catalyseur d'hydroformylation est prélevée d'un réacteur d'hydroformylation,
b) le mélange réactionnel liquide prélevé est transféré dans un récipient de préformage,
c) le mélange réactionnel liquide dans le récipient de préformage est mis en contact avec du monoxyde de carbone, et
d) le mélange réactionnel liquide est renvoyé dans le réacteur d'hydroformylation.

2. Procédé d'hydroformylation selon la revendication 1, dans lequel la pression et/ou la température dans le récipient de préformage et la pression et/ou la température dans ledit au moins un réacteur d'hydroformylation sont différentes.

3. Procédé d'hydroformylation selon la revendication 2, dans lequel la pression et la température du mélange réactionnel liquide lors du transfert dans le récipient de préformage et/ou lors du renvoi dans le réacteur d'hydroformylation sont chaque fois adaptées.

4. Procédé d'hydroformylation selon l'une quelconque des revendications précédentes, dans lequel au moins une partie de la phase gazeuse provenant du récipient de préformage est prélevée et renvoyée au réacteur d'hydroformylation.

5. Procédé d'hydroformylation selon la revendication 4, dans lequel la phase gazeuse prélevée est réunie avec du gaz de synthèse, avant d'être envoyée au réacteur d'hydroformylation.

6. Procédé d'hydroformylation selon l'une quelconque des revendications précédentes, dans lequel le catalyseur d'hydroformylation comprend du Co ou du Rh, de préférence du Co, et un ligand phosphoré.

7. Procédé d'hydroformylation selon la revendication 6, dans lequel le catalyseur d'hydroformylation comprend du Co et un ligand phosphoré.

8. Procédé d'hydroformylation selon l'une quelconque des revendications précédentes, dans lequel la température lors de l'hydroformylation est dans la plage de 70 à 250 C.

9. Procédé d'hydroformylation selon l'une quelconque des revendications précédentes, dans lequel la pression lors de l'hydroformylation se situe dans la plage de 20 à 350 bars.

10. Procédé d'hydroformylation selon l'une quelconque des revendications précédentes, dans lequel sont mises à réagir dans le procédé des oléfines ayant de 4 à 16 atomes de carbone.

11. Procédé d'hydroformylation selon la revendication 10, dans lequel sont mises à réagir dans le procédé des oléfines ayant de 4 à 12 atomes de carbone.
